(19) Europäisches Patentamt
European Patent Office
Office européen des brevets

(11) EP 1 430 886 A1

(12) **DEMANDE DE BREVET EUROPEEN**

(43) Date de publication:
**23.06.2004 Bulletin 2004/26**

(51) Int Cl.[7]: **A61K 7/50**, A61K 7/075

(21) Numéro de dépôt: **03292897.0**

(22) Date de dépôt: **21.11.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **19.12.2002 FR 0216198**

(71) Demandeur: **L'OREAL**
**75008 Paris (FR)**

(72) Inventeur: **Maubru, Mireille**
**78400 Chatou (FR)**

(74) Mandataire: **Le Blainvaux Bellegarde, Françoise**
**L'OREAL - D.I.P.I.**
**25-29 Quai Aulagnier**
**92600 Asnières (FR)**

(54) **Composition de lavage des matières kératiniques à base de tensioactif amphotère et de PEG modifié par un groupe hydrophobe**

(57) L'invention concerne des compositions de nouvelles compositions cosmétiques comprenant dans un milieu cosmétiquement acceptable au moins un tensioactif amphotère choisi parmi les composés alcoylamphohydroxyalkylsulfonates et leurs sels, et au moins un ester ou un éther de polyéthylèneglycol comprenant au moins un groupement hydrophobe en C8-C40.
Ces compositions sont utilisées notamment pour le lavage et/ou le conditionnement des matières kératiniques telles que les cheveux ou la peau.

EP 1 430 886 A1

## Description

La présente invention est relative à des compositions de lavage des matières kératiniques, et en particulier des cheveux et/ou de la peau, comprenant un tensioactif amphotère particulier et un dérivé de polyéthylène glycol modifié par un groupement hydrophobe et au procédé de lavage mettant en oeuvre ces compositions.

Il est connu d'utiliser des tensio-actifs non ioniques, tels que les esters polyoxyéthylénés dans des shampooings. Ces esters de PEG sont utilisés comme épaississants dans ce type de formulation.

Le brevet EP900261 décrit des compositions détergentes et shampooings à base d'associations d'esters éthoxylés peu irritants pour les yeux et ayant un pouvoir moussant satisfaisant.

Dans ce brevet, les compositions détergentes contiennent obligatoirement au moins un dérivé polyoxyéthylénique hydrophobe comme agent tensio-actif non ionique, un agent tensio-actif anionique et une bétaïne tensio-active. C'est l'association de ces trois composés qui confèrent à la composition une mousse d'une bonne stabilité peu irritante pour les yeux.

Les tensioactifs amphotères alcoylamphohydroxyalkylsulfonates ont déjà été préconisés dans des compositions cosmétiques détergentes. Ils ont été décrits par exemple dans la demande de brevet WO99/36054.

Ces compositions de l'art antérieur présentent la caractéristique de ne pas avoir des propriétés cosmétiques satisfaisantes.

L'invention a donc pour but de proposer des compositions cosmétiques détergentes présentant des propriétés cosmétiques améliorées, en particulier le démêlage, le lissage, la souplesse, la malléabilité et la douceur des cheveux.

La demanderesse a découvert, de façon surprenante, qu'il était possible de formuler des compositions cosmétiques notamment de lavage des matières kératiniques, notamment des shampooings présentant une bonne tolérance oculaire et possédant des propriétés cosmétiques améliorées en utilisant un ester ou un éther de polyéthylèneglycol comprenant au moins un groupement hydrophobe et un tensioactif amphotère alcoylamphohydroxyalkylsulfonate.

L'invention a donc pour objet des compositions de lavage des matières kératiniques comprenant au moins un tensio-actif amphotère alcoylamphohydroxyalkylsulfonate et au moins un ester ou un éther de polyéthylèneglycol comprenant au moins un groupement hydrophobe en C8-C 40.

L'invention a encore pour objet l'utilisation d'une composition selon l'invention pour améliorer le démêlage ou le lissage des cheveux, ou pour apporter du volume, de la légèreté, de la douceur, de la souplesse ou de la malléabilité aux cheveux.

L'invention a encore pour objet l'utilisation d'un tensioactif amphotère alcoylampho hydroxyalkyl sulfonate ou ses sels dans une composition cosmétique contenant au moins un ester ou un éther de polyéthylèneglycol comprenant au moins un groupement hydrophobe en C8-C 40 pour améliorer le démêlage ou le lissage des cheveux, ou pour apporter du volume, de la légèreté, de la douceur, de la souplesse ou de la malléabilité aux cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Un autre objet de l'invention est un procédé de lavage des matières kératiniques.

D'autres objets de l'invention apparaîtront à la lecture de la description et des exemples qui suivent.

Selon la présente invention, par matières kératiniques, on comprend les cheveux, les cils, les sourcils, la peau, les ongles, les muqueuses ou le cuir chevelu et plus particulièrement les cheveux. Selon la présente invention, par fibres kératiniques, on comprend les cheveux, les cils, les sourcils, et plus particulièrement les cheveux.

Selon la présente invention, on comprend par groupe ou groupement hydrophobe un groupe ou groupement comportant de 8 à 40 atome de carbone.

Les tensioactifs amphotères alcoylamphohydroxyalkylsulfonates et leurs sels peuvent avoir la formule suivante (I) :

$$R-\underset{\underset{O}{\|}}{C}-NH-A-\overset{\overset{R1}{/}}{N}-A_1-\overset{\overset{OH}{/}}{C}\diagdown A_2-SO_3X$$

dans laquelle :

R désigne un radical hydrocarboné, saturé ou insaturé, linéaire ou ramifié comportant de 5 à 29 atomes de carbone.

R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

R1 désigne un radical hydroxyalkyle en $C_1$-$C_4$, de préférence hydroxyéthyle,

A, A1, A2 , identiques ou différents, désignent un radical alkylène divalent linéaire ou ramifié en $C_1$-$C_{10}$, de préférence en $C_1$-$C_3$

X désigne un atome d'hydrogène, un cation minéral ou organique tel que:

celui d'un métal alcalin (par exemple $Na^+$, $K^+$) , celui d'un métal alcalinoterreux ($Mg^{2+}$, $Ca^{2+}$) , l'ion $NH_4^+$ , les ions ammoniums issus des aminoacides basiques ou des amino-alcools.

Des composés préférés selon la présente invention sont des composés de formule (I) dans laquelle R désigne plus particulièrement un radical alkyle saturé, linéaire ou ramifié comportant de 7 à 29 atomes de carbone, de préférence de 7 à 22 atomes de carbone.

Lorsque X désigne un ion ammonium issu d'alcanolamine, celle-ci peut être la monoéthanolamine, la diéthanolamine, la triéthanolamine, l'amino-3 propanediol-1,2. Lorsque X désigne un ion ammonium issu d'amine, cette amine peut être un aminoacide basique tel que la lysine, l'arginine, la sarcosine, l'ornithine ou la citrulline.

De préférence, A= A2 et désigne -$CH_2CH_2$-. De préférence, A1 désigne-$CH_2$-. De préférence X désigne $Na^+$

Parmi les tensioactifs de formule (I), on peut citer plus particulièrement les sels de Cocoyl amphohydroxypropyl sulfonate et notamment le sel de sodium tel que le produit proposé sous la dénomination MIRANOL CSE par la société RHODIA CHIMIE ou encore les sels de palmytoyl amphohydroxypropyl sulfonate.

Selon l'invention, le ou les tensioactifs amphotères choisis parmi les composés alcoylamphohydroxyalkylsulfonates et leurs sels peuvent représenter de 0,1 % à 30 % en poids, de préférence de 1 % à 20 % en poids, et plus particulièrement 1,5% à 15 par rapport au poids total de la composition finale.

Les esters ou éthers de PEG comprenant au moins un groupement hydrophobe sont par exemple choisis parmi les composés de formules (II) et (III) suivantes :

$$R_2\text{-}(O\text{-}CH_2\text{-}CH_2)_n\text{ - }OR_3 \qquad (II)$$

$$R_2\text{-}(O\text{-}CH_2\text{-}CH_2)_m\text{ - }OH \qquad (III)$$

dans lesquelles :

$R_2$ désigne un groupement alkyle en $C_8$-$C_{40}$, acyle en $C_8$-$C_{40}$, alkylaryle ou aralkyle ayant de 8 à 40 atomes de carbone, linéaire ou ramifié, saturé ou insaturé, un groupement glycéryle, alkyl($C_8$-$C_{40}$)glycéryle, un groupement acyle dérivé d'huile ou de graisse de lanoline, d'huile de noix de coco, d'huile de jojoba, d'huile de castor, d'huile d'amande douce, d'huile d'arachide, d'huile de germe de blé, d'huile de son de riz, d'huile de lin, d'huile de noyaux d'abricot, d'huile de noix, d'huile de palme, d'huile de pistache, d'huile de graines de sésame, d'huile de colza, d'huile de cade, d'huile de mais, d'huile de noyaux de pêche, d'huile de pavot, d'huile de pin, d'huile de soja, d'huile d'avocat, d'huile de graines de tournesol, d'huile de noisette, d'huile d'olive, d'huile de pépins de raisin, d'huile de carthame, de beurre de karité, ou d'huile de babassou.

$R_3$ désigne un atome d'hydrogène, un groupement alkyle ou un groupement acyle ayant de 1 à 40 atomes de carbone, linéaire ou ramifié, saturé ou insaturé,

n et m sont des nombres entier compris entre 3 et 350.

De préférence $R_2$ est un groupement acyle ayant de 12 à 20 atomes de carbone. De préférence $R_3$ est un groupement acyle ayant de 12 à 20 atomes de carbone.

De préférence n est un nombre compris entre 100 et 300.

De préférence, le rapport en poids de la partie hydrophile (-(O-CH2-CH2)nO) sur la partie hydrophobe ($R_2$ et/ou $R_3$) est compris entre 8 et 1000.

On utilise de préférence un composé de formule (II) dans laquelle $R_2$ et $R_3$ désignent un groupement acyle ayant de 12 à 20 atome de carbone et n est compris entre 100 et 300. On peut par exemple citer le distéarate de PEG 150 et le distéarate de PEG 250.

De tels composés sont notamment vendus sous la dénomination Emanon 3299R par la société KAO et sous la dénomination KESSCO PEG 6000 DS par la société AKZO.

Selon l'invention, on peut également utiliser un ester ou éther de formule (IV)

$$\left[\begin{array}{l}—(OCH_2CH_2)_pOR_4 \\ —(OCH_2CH_2)_qOR_4 \\ —(OCH_2CH_2)_rOR_4\end{array}\right. \qquad (IV)$$

dans laquelle $R_4$ désigne un atome d'hydrogène, un groupement alkyle en $C_8$-$C_{40}$, acyle en $C_8$-$C_{40}$, alkylaryle ou aralkyle ayant de 8 à 40 atomes de carbone, linéaire ou ramifié, saturé ou insaturé,

p , q, r, identiques ou différents, peuvent varier de 0 à 350

la somme p + q +r peut varier de 5 à 350

sous réserve qu'au moins un des groupement R4 soit différent d'un atome d'hydrogène.

Les groupements $R_4$ sont notamment des groupement acyles issus des huiles végétales telles que d'huile de noix de coco, d'huile de jojoba, d'huile de castor, d'huile d'amande douce, d'huile d'arachide, d'huile de germe de blé, d'huile de son de riz, d'huile de lin, d'huile de noyaux d'abricot, d'huile de noix, d'huile de palme, d'huile de pistache, d'huile de graines de sésame, d'huile de colza, d'huile de cade, d'huile de mais, d'huile de noyaux de pêche, d'huile de pavot, d'huile de pin, d'huile de soja, d'huile d'avocat, d'huile de graines de tournesol, d'huile de noisette, d'huile d'olive, d'huile de pépins de raisin, d'huile de carthame, de beurre de karité, ou d'huile de babassou.

On utilisera de préférence les glycérides d'huile de palme oxyéthylénés à 200 moles d'oxyde d'éthylène, les mono, di et glycérides d'huile de coprah oxyéthylénés à 30 moles d'oxyde d'éthylène, les mono, di et tri-glycérides d'huile de coprah oxyéthylénés à 7 moles d'oxyde d'éthylène.

Selon la présente invention, l'ester ou l'éther de PEG peut être présent dans la composition cosmétique dans des proportions de 0,1 à 20%, et préférentiellement de 2 à 15% en poids par rapport au poids total de ladite composition.

Les compositions de l'invention contiennent en outre avantageusement au moins un autre agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques ou leurs mélanges.

Les tensioactifs additionnels convenant à la mise en oeuvre de la présente invention sont notamment les suivants :

(i) Tensioactif(s) anionique(s) :

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl ($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl($C_{12}$-$C_{14}$) sulfates de sodium, de magnésium ou d'ammonium, les alkyl ($C_{12}$-$C_{14}$)éthersulfates de sodium, de magnésium, de triéthanolamine ou d'ammonium oxyéthylénés de 2 à 5 moles d'oxyde d'éthylène (en particulier de 2 à 3), le cocoyl iséthionate de sodium et leurs mélange.

Parmi les tensioactifs anioniques, on préfère utiliser selon l'invention les sels d'alkylsulfates et d'alkyléthersufates et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères additionnels, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.
Parmi les dérivés d'amines, on peut citer les produits commercialisés sous les dénomination MIRANOL, tels que décrits dans les brevets US-2 528 378 et US-2 781 354 et de structures :

$$R_2\text{-}CONHCH_2CH_2\text{-}N(R_3)(R_4)(CH_2COO\text{-}) \quad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-}CONHCH_2CH_2\text{-}N(B)(C) \quad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence au moins un tensioactif anionique ou non ionique, des mélanges d'agents tensioactifs et en particulier des mélanges d'agents tensioactifs anioniques et des mélanges d'agents tensioactifs anioniques et d'agents tensioactifs non ioniques.

Les compositions peuvent également contenir des agents tensio-actifs cationiques utilisés de préférence

dans des proportions comprises entre 0,001 à 5% en poids, par rapport au poids total de la composition.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par exemple chlorures) de tétraalkylammonium comme, par exemple, les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyl diméthyl (myristyl acétate) ammonium, commercialisés sous la dénomination de "CEPHARYL 70" par la Société VAN DYK.

On peut également utiliser les sels (chlorures ou méthylsulfate, notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium, et leurs mélanges. Les radicaux acyle proviennent plus particulièrement d'une huile végétale comme l'huile de palme ou de tournesol.

La concentration des tensio-actifs additionnels autres que les tensioactifs anioniques, peut varier de 0 à 30% et de préférence de 0,5 à 15% en poids, par rapport au poids total de la composition.

La quantité totale de tensioactifs peut varier de 3,5 à 55% en poids et de préférence de 7 à 30% en poids par rapport au poids total de la composition.

Les compositions selon l'invention peuvent contenir également des silicones, à savoir des huiles de polyorganosiloxanes, ou des gommes ou des résines de polyorganosiloxanes, telles que sous la forme de solutions dans des solvants organiques ou encore sous forme d'émulsions ou de microémulsions.

Parmi les polyorganosiloxanes pouvant être utilisés conformément à la présente invention, on peut citer à titre non limitatif :

- les silicones volatiles: celles-ci possèdent un point d'ébullition compris entre 60°C et 260°C. Elles sont choisies parmi les silicones cycliques contenant de 3 à 7 atomes de silicium et de préférence 4 à 5.
- les silicones non volatiles : elles sont constituées principalement par les polyalkylsiloxanes, les polyarylsiloxanes, les polyalkylarylsiloxanes, les gommes de silicone, les résines de silicone ou leurs mélanges et les silicones organomodifiées.

On utilise plus particulièrement les polydiméthylsiloxanes, les silicones aminées et les silicones oxyalkylénées. Les polyorgano-siloxanes peuvent être utilisés dans les compositions de l'invention dans des proportions comprises entre 0,01 et 20% en poids et de préférence entre 0,1 et 10% en poids par rapport au poids total de la composition.

Les compositions de l'invention peuvent contenir en outre un polymère cationique. Selon un mode de réalisation préféré, la composition comprend en outre au moins un polymère cationique.
De manière encore plus générale, au sens de la présente invention, l'expression "polymère cationique" désigne tout polymère contenant des groupements cationiques et/ou des groupements ionisables en groupements cationiques.
Les polymères cationiques utilisables conformément à la présente invention peuvent être choisis parmi tous ceux déjà connus en soi comme améliorant les propriétés cosmétiques, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

(V), (VI)

(VII) (VIII)

dans lesquelles:

$R_1$ et $R_2$ , identiques ou différents, représentent hydrogène ou un groupe alkyle ayant de 1 à 6 atomes de carbone et de préférence méthyle ou éthyle;
$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
X- désigne un anion dérivé d'un acide minéral ou organique tel que un anion méthylsulfate, éthylsulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA ,
- le copolymère d'acrylamide et de méthosulfate de méthacryloyloxyéthyltriméthylammonium vendu sous la

dénomination RETEN par la société HERCULES,

- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT® 734" ou "GAFQUAT® 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX® VC 713 par la société ISP,
- les copolymères vinylpyrrolidone / méthacrylamidopropyl dimethylamine commercialisés notamment sous la dénomination STYLEZE® CC 10 par ISP.
- et les copolymères vinylpyrrolidone / méthacrylamide de diméthylaminopropyle quaternisé tel que le produit vendu sous la dénomination "GAFQUAT® HS 100" par la société ISP.

2) Les polysaccharides cationiques notamment les celluloses, les amidons et les gommes de galactomannanes cationiques. Parmi les polysaccharides cationiques, on peut citer plus particulièrement les éthers de cellulose comportant des groupements ammonium quaternaires, les copolymères de cellulose cationiques ou les celluloses greffées avec un monomère hydrosoluble d'ammonium quaternaire et les gommes de galactomannanes cationiques. Les éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la société AMERCHOL. Ces polymères sont également définis dans le dictionnaire CTFA comme des ammonium quaternaires d'hydroxyéthylcellulose ayant réagi avec un époxyde substitué par un groupement triméthylammonium.
Les copolymères de cellulose cationiques ou les celluloses greffées avec un monomère hydrosoluble d'ammonium quaternaire, sont décrits notamment dans le brevet US 4 131 576. On peut notamment citer les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les produits commercialisés répondant à cette définition sont plus particulièrement les produits vendus sous la dénomination "CELQUAT® L 200" et "CELQUAT® H 100" par la société NATIONAL STARCH.
Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR® C13 S, JAGUAR® C 15, JAGUAR® C 17 ou JAGUAR® C162 par la société RHODIA CHIMIE.
On utilise également des amidons modifiées par un sel (par ex. chlorure) de 2,3-époxypropyl triméthylammonium tels que par exemple le produit dénommé Starch hydroxypropyltrimonium chloride selon la nomenclature INCI et commercialisé sous la dénomination SENSOMER Cl-50 d'ONDEO.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français 2.162.025 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces polyaminoamides, on peut citer plus particulièrement les polymères acide adipique/diméthylaminohydroxypropyl/diéthylène triamine vendus sous la dénomination "CARTARETINE® F, F4 ou F8" par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "HERCOSETT® 57" par la société HERCULES Inc. ou bien sous la dénomination de "PD 170" ou "DELSETTE® 101" par la société HERCULES dans le cas du copolymère d'acide adipique/époxypropyl/diéthylène-triamine.

(7) les cyclopolymères cationiques notamment d'alkyl diallyl amine ou de dialkyl diallyl ammonium tels que les homopolymères ou copolymères comportant comme motif principal de la chaîne des motifs répondant aux formules (IX) ou (X) :

(IX): $-(CH_2)_t-CR_{12}$, $(CH_2)_k$, $R_{12}$, $CH_2-$, $CH_2$, $CH_2$, $N^+$, $R_{10}$, $R_{11}$, $Y^-$

(X): $-(CH_2)_t-C$, $R_{12}$, $(CH_2)_k$, $C$, $R_{12}$, $CH_2-$, $CH_2$, $CH_2$, $N$, $R_{10}$

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; Y est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

$R_{10}$ et $R_{11}$, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT® 100" par la société NALCO (et ses homologues de faibles masses moléculaires moyenne en poids) et les copolymères de chlorure de diallyldiméthylammonium et d'acrylamide commercialisés sous la dénomination "MERQUAT® 550".

(8) les polymères de diammonium quaternaires contenant des motifs récurrents répondant à la formule :

$$-\overset{R_{13}}{\underset{R_{14}}{N^+}}-A_1-\overset{R_{15}}{\underset{R_{16}}{N^+}}-B_1- \quad X^- \quad X^- \qquad (XI)$$

formule (XI) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou -CO-O-$R_{17}$-D ou - CO-NH-$R_{17}$-D où $R_{17}$ est un alkylène et D un groupement ammonium quaternaire ;

A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

$X^-$ désigne un anion dérivé d'un acide minéral ou organique;

A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n- dans lequel n est compris entre 1 et 100 et de préférence entre 1 et 50, et

dans lequel D désigne :

a) un reste de glycol de formule : -O-Z-O-, où Z désigne un radical hydrocarboné linéaire ou ramifié ou un groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;

c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule : -NH-CO-NH- ;

De préférence, $X^-$ est un anion minéral ou organique monovalent tel qu'un halogénure (chlorure, bromure), un sulfate, ou un carboxylate (acétate, lactate, citrate).

Ces polymères ont une masse moléculaire moyenne en nombre généralement comprise entre 1.000 et 100.000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule :

$$-\overset{R_1}{\underset{R_2}{\overset{|}{\underset{|}{N^+}}}}(CH_2)_n-\overset{R_3}{\underset{R_4}{\overset{|}{\underset{|}{N^+}}}}(CH_2)_p- \quad X^- \; X^- \qquad (XII)$$

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.
Un composé de formule (XII) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(9) les polymères de polyammonium quaternaires constitués de motifs de formule (XIII):

$$-\underset{\underset{R_{19}}{|}}{\overset{\overset{R_{18}}{|}}{N^+}}(X^-)-(CH_2)_r-NH-CO-(CH_2)_q-CO-NH-(CH_2)_s-\underset{\underset{R_{21}}{|}}{\overset{\overset{R_{20}}{|}}{N^+}}(X^-)-A- \quad (XIII)$$

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle

ou $-CH_2CH_2(OCH_2CH_2)_pOH$,
où p est égal à 0 ou à un nombre entier allant de 1 et 6, sous réserve que $R_{18}$, $R_{19}$, $R_{20}$ et $R_{21}$ ne représentent pas simultanément un atome d'hydrogène,

r et s, identiques ou différents, sont des nombres entiers allant de 1 et 6,
q est égal à 0 ou à un nombre entier allant de 1 et 34,
$X^-$ désigne un anion tel qu'un halogènure,
A désigne un radical d'un dihalogénure ou représente de préférence

$$-CH_2-CH_2-O-CH_2-CH_2-.$$

De tels composés sont notamment décrits dans la demande de brevet EP-A-122 324.
On peut par exemple citer parmi ceux-ci, les produits "MIRAPOL® A 15", "MIRAPOL® AD1", "MIRAPOL®AZ1" et "MIRAPOL® 175" vendus par la société MIRANOL.

(11) Les polymères quaternaires de vinylpyrrolidone et de vinylimidazole tels que les polyquaternium-11, polyquaternium-16, polyquaternium-44 notamment les produits commercialisés sous les dénominations LUVIQUAT® FC 905, FC 550, FC 370 et LUVIQUAT® CARE par la société B.A.S.F.

(11) Les polyamines comme le POLYQUART® H vendu par COGNIS, référencé sous le nom de « POLYETHYLENEGLYCOL (15) TALLOW POLYAMINE » dans le dictionnaire CTFA.

(12) Les polymères réticulés ou non réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la société CIBA.
D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les éthers de cellulose comportant des goupements ammonium quaternaires tels que les produits commercialisés sous la dénomination « JR 400 » par la société AMERCHOL, les cyclopolymères cationiques, en particulier les homopolymères de sel de diallyldiméthylammonium et les copolymères de sel de diallyldiméthylammonium et d'acrylamide en particulier les chlorures, commercialisés sous les dénominations « MERQUAT® 100 », « MERQUAT® 550 » et « MERQUAT® S » par la société NALCO, les gommes de guar modifiées par du chlorure de 2,3-époxypropyl triméthylammonium commercialisées par exemple sous la dénomination « JAGUAR C13S » par la société RHODIA et leurs mélanges.

Selon l'invention, le ou les polymères cationiques peuvent représenter de 0,001 % à 20 % en poids, de préférence de 0,01 % à 10% en poids et plus particulièrement de 0,05 à 3% en poids par rapport au poids total de la composition finale.

Les compositions, selon l'invention, présentent un pH généralement compris entre 3 et 12, et plus particulièrement entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Le milieu cosmétiquement acceptable peut être constitué uniquement par de l'eau ou par un mélange d'eau et d'un solvant cosmétiquement acceptable tel qu'un alcool inférieur en $C_1$-$C_4$, comme l'éthanol, l'isopropanol, le tertiobutanol, le n-butanol ; les alkylèneglycols comme le propylèneglycol, les éthers de glycols.

De préférence , la composition comprend de 50 à 95 % en poids d'eau par rapport au poids total de la composition.

Les compositions selon l'invention peuvent contenir également des agents régulateurs de viscosité, tels que des électrolytes comme le chlorure de sodium, des épaississants comme les dérivés de la cellulose, tels que par exemple la carboxyméthylcellulose, l'hydroxypropyl- cellulose, l'hydroxyéthylcellulose, la gomme de guar, les gommes de guar hydroxypropylées, les scléroglucanes, la gomme de xanthane, les polymères amphiphiles comportant au moins une chaîne grasse.

Les compositions selon l'invention peuvent également contenir différents adjuvants habituellement utilisés en cosmétique, tels que des parfums, des conservateurs, des synergistes de mousse, des séquestrants, des stabilisateurs de mousse, des agents propulseurs, des colorants, des polymères notamment amphotères, des agents anti-pelliculaires, des céramides, des pseudocéramides, des vitamines ou provitamines, des hydroxy acides, des agents acidifiants ou alcalinisants, des huiles végétales minérales, animales, des huiles de synthèse telles que les polyisobutènes et les polydécènes, des esters d'acides gras non polyoxyéthylénés, des agents nacrants ou d'autres adjuvants selon l'usage envisagé.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en $C_{10}$-$C_{30}$ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants et/ou opacifiants bien connus dans l'état de la technique tels que par exemple les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C 40) tels que par exemple le distéaryléther ou le 1-(hexadécyloxy)-2-octadécanol, les cyclodextrines, les alcools gras tels que les alcools cétylique, stéarylique et béhénylique.

L'invention a aussi pour objet l'utilisation comme shampooing de la composition sus définie.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Un autre objet de la présente invention est un procédé de lavage des matières kératiniques, qui consiste dans l'application sur ces matières d'au moins une composition de la présente invention, suivie d'un rinçage des matières traitées après un éventuel temps de pose.

Les exemples qui suivent sont destinés à illustrer l'invention sans pour autant présenter un caractère limitatif.

Exemples 1 à 3:

On a réalisé les compositions de shampooing suivantes :

| Composition | Exemple 1 | Exemple 2 | Exemple 3 |
|---|---|---|---|
| Lauryléthersulfate de sodium(C12/C14 à 70/30) à 2.2 moles d'oxyde d'éthylène | 7 g MA | 7 g MA | 7 g MA |
| Cocoyl amphohydroxypropyl sulfonate de sodium, en solution aqueuse à 32% de matière active, proposé sous la dénomination Miranol CSE par la société RHODIA | 3.8 g MA | 3.8 g MA | 3.2 g MA |
| Mélange glycéride de palme oxyéthyléné (200 OE) et glycéride de coprah oxyéthyléné (7 OE) en suspension aqueuse à 70% | 7 g | 7 g | 0.5 g |
| Mono/diglycérides de coprah oxyéthyléné (30 OE) | 1 g | - | - |
| Mono-laurate de sorbitan 4 OE | - | 0.5 g | 7 g |
| Mono-laurate de sorbitan 20 OE | - | 2.5 g | 1 g |
| Hydroxyéthylcellulose réticulée par l'épichlorhydrine et quaternisée par la triméthylamine, vendue sous la dénomination JR 400 par la société AMERCHOL | 0.25 g | 0.25 g | 0.25 g |
| Conservateurs | q.s. | q.s. | q.s. |
| Acide citrique q.s. | pH 7 | pH 5.3 | pH 7 |
| Eau déminéralisée q.s.p. | 100 g | 100 g | 100 g |

Les cheveux lavés avec ces compositions sont lisses et se démêlent facilement.

EXEMPLE 4

On a préparé la composition de shampooing suivante :

| | |
|---|---|
| Lauryléthersulfate de sodium (C12/C14 à 70/30) à 2.2 moles d'oxyde d'éthylène | 4.5 g M.A. |
| Cocoyl amphohydroxypropyl sulfonate de sodium, en solution aqueuse à 38% de matière active, commercialisé sous la dénomination Miranol CS par la société RHODIA | 2.3 g M.A. |
| Mélange glycérides de palme oxyéthyléné (200 OE) et de coprah oxyéthyléné (7 OE) en suspension aqueuse à 70% | 4.8 g |
| Hydroxyéthylcellulose réticulée par l'épichlorhydrine et quaternisée par la triméthylamine, vendue sous la dénomination JR 400 par la société AMERCHOL | 0.2 g |
| Conservateurs, parfum . | q.s. |
| Acide citrique q.s.p. | pH 5.3 |
| Eau déminéralisée q.s.p. | 100 g |

**Revendications**

1. Composition cosmétique, **caractérisée par le fait qu'**elle comprend, dans un milieu cosmétiquement acceptable, au moins un tensioactif amphotère choisi parmi les composés alcoylamphohydroxyalkylsulfonates et leurs sels et au moins un ester ou un éther de polyéthylèneglycol comprenant au moins un groupement hydrophobe en $C_8$-$C_{40}$.

2. Composition selon la revendication 1, **caractérisée par le fait que** ledit tensioactif amphotère est choisi parmi les composés alcoylamphohydroxyalkylsulfonates ayant la formule suivante (I) :

$$R-\underset{\underset{O}{\|}}{C}-NH-A-\overset{\overset{R1}{|}}{N}-A_1-\overset{\overset{OH}{/}}{C}\diagdown A_2-SO_3X$$

dans laquelle :

R désigne un radical hydrocarboné, saturé ou insaturé, linéaire ou ramifié comportant de 5 à 29 atomes de carbone.
R1 désigne un radical hydroxyalkyle en $C_1$-$C_4$, de préféérence hydroxyéthyle,
A, A1, A2 , identiques ou différents, désignent un radical alkylène divalent linéaire ou ramifié en $C_1$-$C_{10}$, de préférence en $C_1$-$C_3$
X désigne un atome d'hydrogène, un cation minéral ou organique.

3. Composition selon la revendication 2, **caractérisée par le fait que** R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

4. Composition selon l'une quelconque des revendications 2 ou 3, **caractérisée par le fait que** A et $A_2$ désignent $-CH_2CH_2-$.

5. Composition selon l'une quelconque des revendications 2 à 4, **caractérisée par le fait que** $A_1$ désigne $-CH_2-$.

6. Composition selon l'une quelconque des revendications 2 à 5, **caractérisée par le fait que** le cation minéral ou organique est choisi parmi celui d'un métal alcalin ( par exemple $Na^+$, $K^+$) , celui d'un métal alcalinoterreux ($Mg^{2+}$, $Ca^{2+}$) , l'ion $NH_4^+$ , les ions ammoniums issus des aminoacides basiques ou des amino-alcools.

7. Composition selon l'une quelconque des revendications 1 à 6, **caractérisée par le fait que** ledit tensioactif amphotère est choisi parmi les sels de Cocoyl amphohydroxypropyl sulfonate et les sel de palmytoyl amphohydroxypropyl sulfonate.

8. Composition selon l'une quelconque des revendications 1 à 7, **caractérisée en ce que** le tensioactif amphotère est présent dans les compositions à une concentration comprise entre 0,1 et 30 % en poids, de préférence entre 1,5 et 20% en poids par rapport au poids total de la composition.

9. Composition selon l'une quelconque des revendications 1 à 8, **caractérisée en ce que** l'ester ou l'éther de polyéthylèneglycol comprenant au moins un groupement hydrophobe en C8-C40 est choisi parmi les composés de formules (II) et (III) suivantes :

$$R_2\text{-}(O\text{-}CH_2\text{-}CH_2)_n\text{ - }OR_3 \qquad \text{(II)}$$

$$R_2\text{-}(O\text{-}CH_2\text{-}CH_2)_m\text{ - }OH \qquad \text{(III)}$$

dans lesquelles :

$R_2$ désigne un groupement alkyle en $C_8$-$C_{40}$, acyle en $C_8$-$C_{40}$, alkylaryle ou aralkyle ayant de 8 à 40 atomes de carbone, linéaire ou ramifié, saturé ou insaturé, un groupement glycéryle, alkyl($C_8$-$C_{40}$)glycéryle, un groupement acyle dérivé d'huile ou de graisse de lanoline, d'huile de noix de coco, d'huile de jojoba, d'huile de castor, d'huile d'amande douce, d'huile d'arachide, d'huile de germe de blé, d'huile de son de riz, d'huile de lin, d'huile de noyaux d'abricot, d'huile de noix, d'huile de palme, d'huile de pistache, d'huile de graines de sésame, d'huile de colza, d'huile de cade, d'huile de mais, d'huile de noyaux de pêche, d'huile de pavot, d'huile de pin, d'huile de soja, d'huile d'avocat, d'huile de graines de tournesol, d'huile de noisette, d'huile d'olive, d'huile de pépins de raisin, d'huile de carthame, de beurre de karité, ou d'huile de babassou,

$R_3$ désigne un atome d'hydrogène, un groupement alkyle ou un groupement acyle ayant de 1 à 40 atomes de carbone, linéaire ou ramifié, saturé ou insaturé,

n et m sont des nombres entier compris entre 3 et 350.

10. Composition selon la revendication 9, **caractérisée en ce que** $R_2$ est un groupement acyle ayant de 12 à 20

atomes de carbone.

**11.** Composition selon l'une quelconque des revendications 9 ou 10, **caractérisée en ce que** $R_3$ est un groupement acyle ayant de 12 à 20 atomes de carbone.

**12.** Composition selon l'une quelconque des revendications 9 à 11, **caractérisée en ce que** n est un nombre compris entre 100 et 300.

**13.** Composition selon l'une quelconque des revendications 1 à 8, **caractérisée en ce que** l'ester ou l'éther de polyéthylèneglycol comprenant au moins un groupement hydrophobe en C8-C40 est choisi parmi les composés de formules (IV) suivante :

$$\left[\begin{array}{l} -(OCH_2CH_2)_pOR_4 \\ -(OCH_2CH_2)_qOR_4 \\ -(OCH_2CH_2)_rOR_4 \end{array}\right. \qquad (IV)$$

dans laquelle $R_4$ désigne un atome d'hydrogène, un groupement alkyle en $C_8$-$C_{40}$, acyle en $C_8$-$C_{40}$, alkylaryle ou aralkyle ayant de 8 à 40 atomes de carbone, linéaire ou ramifié, saturé ou insaturé, des groupement acyles issus d'huiles végétales,

p , q, r, identiques ou différents, varient de 0 à 350

la somme p + q +r varie de 5 à 350

sous réserve qu'au moins un des groupement $R_4$ soit différent d'un atome d'hydrogène.

**14.** Composition selon la revendication 13, **caractérisée en ce que** les groupements $R_4$ sont notamment des groupement acyles issus des huiles végétales telles que d'huile de noix de coco, d'huile de jojoba, d'huile de castor, d'huile d'amande douce, d'huile d'arachide, d'huile de germe de blé, d'huile de son de riz, d'huile de lin, d'huile de noyaux d'abricot, d'huile de noix, d'huile de palme, d'huile de pistache, d'huile de graines de sésame, d'huile de colza, d'huile de cade, d'huile de mais, d'huile de noyaux de pêche, d'huile de pavot, d'huile de pin, d'huile de soja, d'huile d'avocat, d'huile de graines de tournesol, d'huile de noisette, d'huile d'olive, d'huile de pépins de raisin, d'huile de carthame, de beurre de karité, ou d'huile de babassou.

**15.** Composition selon l'une quelconque des revendications 13 ou 14, **caractérisée en ce que** l'ester ou l'éther de polyéthylèneglycol comprenant au moins un groupement hydrophobe en $C_8$-$C_{40}$ est choisi parmi les glycérides d'huile de palme oxyéthylénés à 200 moles d'oxyde d'éthylène, les mono, di et glycérides d'huile de coprah oxyéthylénés à 30 moles d'oxyde d'éthylène, les mono, di et tri-glycérides d'huile de coprah oxyéthylénés à 7 moles d'oxyde d'éthylène.

**16.** Composition selon l'une quelconque des revendications 1 à 15, **caractérisée en ce que** l'ester ou l'éther de polyéthylèneglycol comprenant au moins un groupement hydrophobe en $C_8$-$C_{40}$ est présent dans des proportions de 0,1 à 20%, et préférentiellement de 2 à 15% en poids par rapport au poids total de ladite composition.

**17.** Composition selon l'une quelconque des revendications précédentes, **caractérisée par le fait qu'**elle contient en outre au moins un tensioactif choisi parmi les tensio-actifs non-ioniques, amphotères ou zwittérioniques différents des tensioactifs amphotères sus-définis.

**18.** Composition selon la revendication 17, **caractérisée en ce que** le ou les tensioactifs additionnels sont présents dans des quantités allant de 0,1 à 60% en poids.

**19.** Composition selon l'une quelconque des revendications précédentes, **caractérisée en ce que** la quantité totale de tensioactifs va de 3,5 à 55% en poids et de préférence de 7 à 30% en poids par rapport au poids total de la composition.

**20.** Composition selon l'une quelconque des revendications précédentes, **caractérisée par le fait qu'**elle contient en

outre un ou plusieurs agents tensio-actifs cationiques.

**21.** Composition selon l'une quelconque des revendications précédentes, **caractérisée par le fait qu'**elle contient en outre une ou plusieurs silicones.

**22.** Composition selon l'une quelconque des revendications précédentes, **caractérisée par le fait qu'**elle contient en outre un ou plusieurs polymères cationiques ou amphotères.

**23.** Composition selon la revendication 22, **caractérisée par le fait que** les polymères cationiques ou amphotères sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

**24.** Composition selon l'une quelconque des revendications 22 ou 23, **caractérisée par le fait que** ledit polymère cationique est choisi parmi les éthers de cellulose comportant des goupements ammonium quaternaires, les homopolymères de sel de diallyldiméthylammonium et les copolymères de sel de diallyldiméthylammonium et d'acrylamide, les gommes de guar modifiées par du chlorure de 2,3-époxypropyl triméthylammonium et leurs mélanges.

**25.** Composition selon l'une quelconque des revendications 22 à 24, **caractérisée en ce que** le polymère cationique ou amphotère est présent à une concentration comprise entre 0,001 % et 20 % en poids par rapport au poids total de la composition, de préférence entre 0,01 % et 10 % en poids.

**26.** Composition selon l'une quelconque des revendications précédentes, **caractérisée par le fait qu'**elle contient en outre des agents régulateurs de viscosité, tels que des électrolytes comme le chlorure de sodium, des épaississants comme les dérivés de la cellulose, la gomme de guar, les gommes de guar hydroxypropylées, les scléroglucanes, la gomme de xanthane et les polymères amphiphiles comportant au moins une chaîne grasse.

**27.** Composition selon l'une quelconque des revendications 1 à 26, **caractérisée par le fait qu'**elle contient en outre un ou plusieurs adjuvants choisis parmi les parfums, les conservateurs, les synergistes de mousse, les séquestrants, les stabilisateurs de mousse, les agents propulseurs, les colorants, les agents anti-pelliculaires, les céramides, les vitamines ou provitamines, les hydroxy-acides, les agents acidifiants ou alcalinisants, les huiles végétales minérales, animales, les huiles de synthèse telles que les polyisobutènes et les polydécènes, les esters d'acides gras non polyoxyéthylénés, les pseudocéramides et les agents nacrants et/ou opacifiants.

**28.** Composition selon la revendication 27, **caractérisée par le fait que** d'agents nacrants et/ou opacifiants sont choisis parmi les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses en C8-C40 tels que par exemple le distéaryléther ou le 1-(hexadécyloxy)-2-octadécanol, les cyclodextrines, les alcools gras tels que les alcools cétylique, stéarylique et béhénylique.

**29.** Utilisation comme shampooing de la composition telle que définie dans l'une quelconque des revendications 1 à 28.

**30.** Utilisation d'une composition selon l'une quelconque des revendications précédentes pour améliorer le démêlage ou le lissage des cheveux, ou pour apporter du volume, de la légèreté, de la douceur, de la souplesse ou de la malléabilité aux cheveux.

**31.** Utilisation d'un tensioactif amphotère alcoylampho hydroxyalkyl sulfonate ou ses sels dans une composition cosmétique contenant au moins un ester ou un éther de polyéthylèneglycol comprenant au moins un groupement hydrophobe en C8-C40 pour améliorer le démêlage ou le lissage des cheveux, ou pour apporter du volume, de la légèreté, de la douceur, de la souplesse, de la malléabilité aux cheveux.

**32.** Procédé de lavage des matières kératiniques, **caractérisé par** l'application sur ces matières d'au moins une composition telle que définie dans l'une quelconque des revendications 1 à 28, suivie d'un rinçage des matières traitées après un éventuel temps de pose.

Office européen des brevets

# RAPPORT DE RECHERCHE EUROPEENNE

Numéro de la demande
EP 03 29 2897

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 2001/009909 A1 (DECOSTER SANDRINE ET AL) 26 juillet 2001 (2001-07-26)<br>* page 1, alinéa 1 - alinéa 2 *<br>* page 2, alinéa 27; revendications; exemple 4 *<br>--- | 1-8, 29-32 | A61K7/50<br>A61K7/075 |
| A,D | US 6 475 474 B1 (RICCA JEAN-MARC) 5 novembre 2002 (2002-11-05)<br>* colonne 1, ligne 25 - ligne 67; revendications; exemple 10 *<br>--- | 1-8, 29-32 | |
| A | WO 98 27938 A (BELLEMAIN CHANTAL MARIE CLAUDE ;DAWSON GEOFFREY GEORGE (GB); PROCT) 2 juillet 1998 (1998-07-02)<br>* page 1 - page 3 *<br>* page 8; revendications; exemples 5-8 *<br>--- | 1,29-32 | |
| A | WO 97 04743 A (COLGATE PALMOLIVE CO) 13 février 1997 (1997-02-13)<br>* page 1, ligne 5 - ligne 16 *<br>* page 2, ligne 31 - page 3, ligne 36 *<br>* page 6, ligne 1 - ligne 10; revendications; exemples *<br>----- | 1,29-32 | |

| DOMAINES TECHNIQUES RECHERCHES (Int.Cl.7) |
|---|
| A61K |

Le présent rapport a été établi pour toutes les revendications

| Lieu de la recherche | Date d'achèvement de la recherche | Examinateur |
|---|---|---|
| MUNICH | 23 mars 2004 | Mellgren, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO. EP 03 29 2897

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-03-2004

| Document brevet cité au rapport de recherche | Date de publication | Membre(s) de la famille de brevet(s) | Date de publication |
|---|---|---|---|
| US 2001009909 A1 | 26-07-2001 | FR 2804020 A1 | 27-07-2001 |
| | | AU 759294 B2 | 10-04-2003 |
| | | AU 1503201 A | 26-07-2001 |
| | | BR 0100135 A | 28-08-2001 |
| | | CA 2332474 A1 | 21-07-2001 |
| | | CN 1306813 A | 08-08-2001 |
| | | CZ 20010207 A3 | 12-09-2001 |
| | | EP 1132079 A1 | 12-09-2001 |
| | | HU 0100271 A2 | 29-05-2002 |
| | | JP 2001206823 A | 31-07-2001 |
| | | PL 345176 A1 | 30-07-2001 |
| | | RU 2203027 C2 | 27-04-2003 |
| | | ZA 200100312 A | 26-07-2001 |
| US 6475474 B1 | 05-11-2002 | FR 2773710 A1 | 23-07-1999 |
| | | AU 2058699 A | 02-08-1999 |
| | | BR 9907113 A | 21-08-2001 |
| | | CA 2318337 A1 | 22-07-1999 |
| | | EP 1049456 A1 | 08-11-2000 |
| | | WO 9936054 A1 | 22-07-1999 |
| | | US 2003086953 A1 | 08-05-2003 |
| WO 9827938 A | 02-07-1998 | AU 5614798 A | 17-07-1998 |
| | | EP 0952809 A1 | 03-11-1999 |
| | | JP 2001507034 T | 29-05-2001 |
| | | WO 9827938 A1 | 02-07-1998 |
| | | ZA 9711424 A | 01-07-1998 |
| WO 9704743 A | 13-02-1997 | AU 699741 B2 | 10-12-1998 |
| | | AU 6679996 A | 26-02-1997 |
| | | BR 9609909 A | 15-06-1999 |
| | | CN 1196675 A ,B | 21-10-1998 |
| | | WO 9704743 A1 | 13-02-1997 |
| | | US 5747435 A | 05-05-1998 |
| | | ZA 9606404 A | 26-01-1998 |

EPO FORM P0460

Pour tout renseignement concernant cette annexe : voir Journal Officiel de l'Office européen des brevets, No.12/82